# FASCICULE DE BREVET EUROPEEN

(11) **EP 1 282 397 B1**
(45) Date de publication et mention de la délivrance du brevet: **04.05.2005**
(21) Numéro de dépôt: 01934114.8
(22) Date de dépôt: 14.05.2001
(51) Int. Cl.: A61K 7/48

(54) **UTILISATION DE COMPOSES CONTENANT UNE FONCTION THIO-ETHER, SULFOXYDE OU SULFONE COMME AGENT COSMETIQUE ANTI-POLLUTION**
VERWENDUNG VON VERBINDUNGEN, DIE EINE THIOETHERFUNKTION, SULFOXIDFUNKTION ODER SULFONFUNKTION ENTHALTEN, ALS KOSMETISCHE WIRKSTOFFE GEGEN IN DER ATMOSPHÄRE VORHANDENE SCHADSTOFFE.
USE OF COMPOUNDS CONTAINING A THIO-ETHER, SULPHOXIDE OR SULPHONE FUNCTION AS COSMETIC ANTI-POLLUTION AGENT

(30) Priorité: 18.05.2000 FR 0006386
(43) Date de publication de la demande: 12.02.2003
(73) Titulaire: L'OREAL, 75008 Paris (FR)
(72) Inventeur: CATROUX, Philippe, F-94130 Nogent-sur-Marne (FR); MAIGNAN, Jean, F-93290 Tremblay-en-France (FR); COTOVIO, José, F-77270 Dammartin-en-Goele (FR)
(74) Mandataire: Casalonga, Axel
(86) Numéro de dépôt international: PCT/FR2001/001453
(87) Numéro de publication internationale: WO 2001/087261

(56) Documents cités:
- WO-A-94/03149
- WO-A-97/16165
- FR-A- 2 476 484
- FR-A- 2 735 977

## Description

La présente invention concerne essentiellement une nouvelle utilisation de composés contenant une fonction thio-éther, sulfoxyde ou sulfone comme agent cosmétique anti-pollution.

Les milieux urbains sont régulièrement soumis à des pics de pollution. Les polluants atmosphériques qui sont représentés largement par les produits primaires et secondaires de la combustion représentent une source importante du stress oxydatif environnemental.

Différents types de produits chimiques, xénobiotiques et particules composent la pollution urbaine.

Trois grandes catégories de polluants peuvent exercer des effets délétères sur la peau et le cheveu: les gaz, les métaux lourds et les particules qui sont les résidus de combustion sur lesquelles sont adsorbés de très nombreux composés organiques.

Ce sont les tissus les plus externes qui sont initialement et directement exposés aux toxiques de l'environnement. La peau est directement et fréquemment exposée à l'environnement pro-oxydant. Les sources d'oxydants environnementaux incluent l'oxygène, l'irradiation solaire UV ainsi que dans l'air pollué, l'ozone et les oxydes d'azote et de soufre. Les polluants atmosphériques représentés par les produits primaires et secondaires des combustions domestiques et industrielles tels que les hydrocarbures aromatiques mono- et polycycliques sont aussi une source importante du stress oxydatif. La peau est particulièrement sensible à l'action du stress oxydatif et la couche la plus externe sert de barrière vis-à-vis des dommages oxydatifs. Dans la plupart des circonstances, l'oxydant a des chances d'être neutralisé après réaction avec les matières kératiniques, mais les produits de réaction formés peuvent être responsables d'atteintes cellulaires et tissulaires.

Le stratum cornéum, la barrière de la peau, est le site de contact entre l'air et le tissu cutané. La structure biphasique lipides/protéines est un déterminant crucial de cette fonction de barrière de la peau. Ces éléments peuvent réagir avec les oxydants et sont altérés ce qui favorise les phénomènes de desquamation. La péroxydation lipidique induite par l'ozone peut altérer la peau de deux façons:
1/ l'oxydation et la dégradation des lipides du stratum coméum peut altérer la fonction barrière du stratum cornéum. La perturbation des lipides externes et de l'architecture des protéines semblent être des facteurs déclenchants dans de nombreuses dermatoses (psoriasis, dermatite atopique, dermatite irritante).
2/ la formation accrue de produits d'oxydation des lipides dans les couches de peau supérieures peut déclencher des atteintes dans les couches cutanées adjacentes. La réaction de l'ozone (O₃) avec les lipides insaturés intervient par des réactions d'addition connues sous le nom de réaction de Criegge selon le schéma décrit ci-dessous:

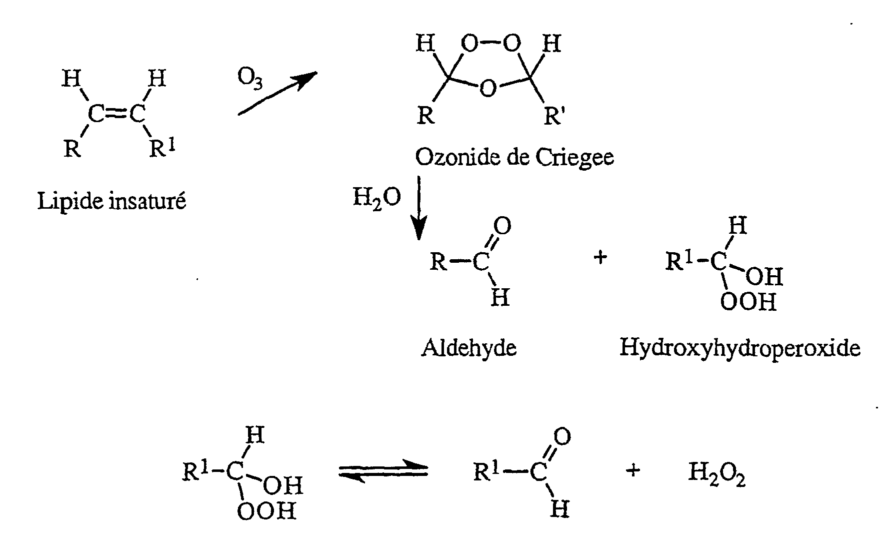

Ce processus conduit dans un deuxième temps à la cassure des chaînes lipidiques et à la formation d'hydroperoxydes et d'aldéhydes ainsi que de peroxyde d'hydrogène. Il s'agit d'un mécanisme spécifique différent du mécanisme de lipoperoxydation classiquement décrit, lequel est médié par un radical. Les produits d'oxydation lipidique secondaires ou tertiaires induits par l'ozone, et qui ont donc une réactivité inférieure à l'ozone mais une durée de vie supérieure, peuvent propager l'effet de l'ozone. Du fait de leur stabilité relative, les produits d'oxydation des lipides et de peroxydation, c'est à dire les aldéhyde et les oxydes de cholestérol, ont le potentiel d'altérer les cellules à des sites distants non directement exposés à l'ozone.

Une atteinte oxydative significative au niveau des couches superficielles du stratum peut initier des processus inflammatoires localisés sous-jacents, conduisant au recrutement de phagocytes qui en générant des oxydants vont amplifier les processus oxydatifs initiaux.

Dans la pollution urbaine, l'exposition concomitante à l'ozone et aux UV peut causer un stress oxydatif synergique.

De même, on peut penser qu'il existe une synergie d'action entre l'ozone et les composés organiques (hydrocarbures aromatiques monocycliques et polycycliques) issus de la combustion.

Des solutions ont déjà été envisagées dans les traitements cosmétiques et thérapeutiques en protégeant les tissus par des composés avec des groupes soufrés qui se comportent comme des séquestrants de métaux lourds, comme les métallothionéines dans le brevet EP 0 557 042 Al et les composés aminoacides avec des groupements thiols dans la demande de brevet EP 0 914 815 A1.

Par ailleurs, les acides carboxyliques porteurs d'une fonction thio-éther ou d'une fonction sulfoxydes sont décrits dans le brevet EP-A-0576287 revendiquant leur capacité à améliorer l'élasticité de la peau.

Il existe des utilisations antérieures des acides carboxyliques porteurs d'une fonction soufrée pour favoriser la desquamation de la peau ou stimuler le renouvellement épidermique.

Le document brevet WO-A-97/16165 décrit l'utilisation de composés de formule (I)-(III) pour stimuler le renouvellement cellulaire et la réparation epidermique .

Le problème posé est donc de protéger la peau contre les gaz toxiques, et plus particulièrement l'ozone, et leurs effets délétères rencontrés dans la pollution urbaine.

Il a maintenant été constaté de manière tout à fait surprenante que l'utilisation en application topique de composés contenant une fonction thio-éther, sulfoxide ou sulfone, permettait de protéger les matières kératiniques, la peau et les phanères, contre les effets délétères des gaz, et plus particulièrement l'ozone.

La demanderesse a découvert que ces composés permettait de préserver et de protéger d'une manière inexplicable les matières kératiniques, la peau et les phanères contre les effets nocifs de la pollution.

Les thio-éthers donnent avec l'ozone des produits d'oxydation stables sous forme de sulfoxydes alors que les sulfoxydes peuvent au contact de l'ozone être transformés en sulfones correspondantes. Ces dernières en présence d'un excès d'ozone se transforment en acide sulfonique correspondants.

La présente invention a pour objet une utilisation en application topique de composés contenant une fonction thio-éther, sulfoxide ou sulfone, comme agents cosmétiques anti-pollution.

On entend par agent cosmétique anti-pollution un agent qui protège la peau et les matières kératiniques de façon à prévenir, atténuer et/ou supprimer les effets délétères des gaz toxiques tels que l'ozone et des composés organiques résidus de combustions.

Ces composés contenant une fonction thio-éther, sulfoxide ou sulfone répondent aux formules I, II et III suivantes : 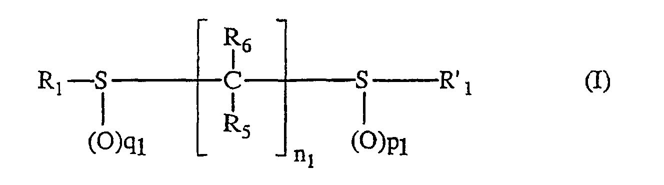 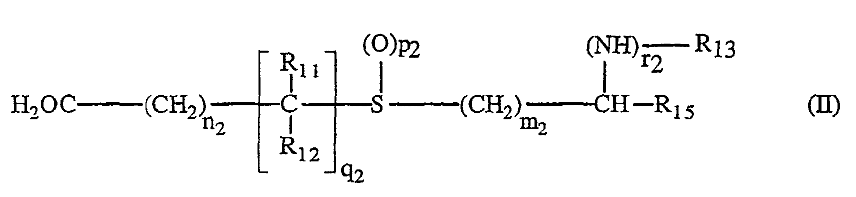 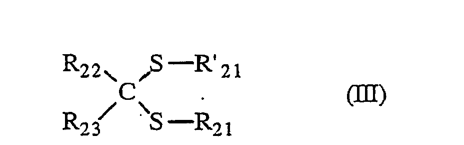 n₁ représente un nombre entier d'une valeur allant de 1 à 10,
p₁ et q₁, identiques ou différents, représentent 0, 1 ou 2,
n₂ représente un nombre entier d'une valeur allant de 0 à 5,
p₂ représente 0, 1 ou 2,
q₂ représente 0, 1, 2 ou 3,
m₂ représente un nombre entier d'une valeur allant de 0 à 10,
r₂ représente 0 ou 1,
q₂ et n₂ n'étant jamais tous deux nuls simultanément.

Les radicaux R₁ et R'₁, identiques ou différents, représentent un radical choisi parmi les groupements:
a) 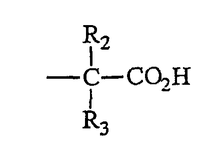
b) 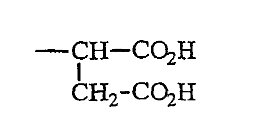
c) 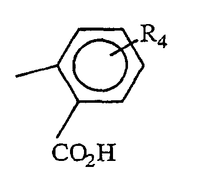
dans lesquels,
R₂ et R₃, identiques ou différents désignent un atome d'hydrogène ou un groupement alcoyle en C₁-C₈ ou alcényle C₂-C₈, linéaires ou ramifiés; R₄ désigne un atome d'hydrogène ou un groupement alcoyle en C₁-C₈ linéaire ou ramifié, un groupement alcényle en C₂-C₈ linéaire ou ramifié, un groupement alcoxy en C₁-C₈ linéaire ou ramifié;
R₅ et R₆ désignent indépendamment l'un de l'autre un atome d'hydrogène, alcényle en C₂-C₈, alcoxy en C₁-C₈ linéaire ou ramifié, phényle, alcoylcarboxylique en C₁-C₆, alcénylcarboxylique en C₂-C₆;
R₁₁ et R₁₂ ont les mêmes significations que R₅ et R₆;
R₁₃ représente un groupement alcoyle en C₁-C₁₇ ou alcényle en C₂-C₁₇ linéaires ou ramifiés ou un radical acyle-CO-R₁₄, avec R₁₄ qui désigne un groupement alcoyle en C₁-C₁₇ ou alcényle en C₂-C₁₇ linéaires ou ramifiés;
R₁₅ est choisi parmi un atome d'hydrogène, un groupement alcoyle en C₁-C₈, alcényle en C₂-C₈ linéaires ou ramifiés, un groupement alcoylcarboxylique en C₁-C₅, alcénylcarboxylique en C₂-C₅, ou la fonction -COOH;
R₂₁ et R'₂₁ désignent les groupements de formule: 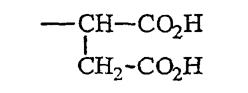 et

―CH₂-CO₂H

R₂₂ représente un groupement:
i)

   ―CH₃
ii)

   ―CO₂H
iii) 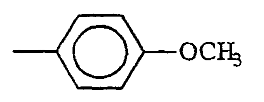
iv) 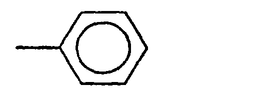
v) 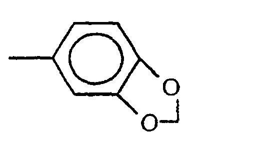
vi) 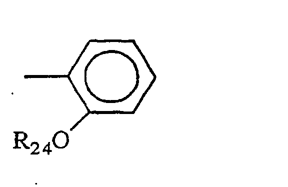
vii) 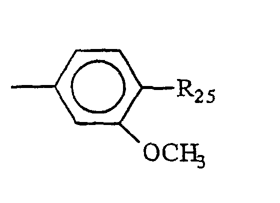
tel que R₂₄ représente un groupement choisi parmi: -CH₂-COOH et -CO-CH₃ et R₂₅ représente un groupement choisi parmi: OH, OCH₃, OC(CH₃)₃, OCH₂COOH;
R₂₃ désigne un atome d'hydrogène, un radical alcoyle en C₁-C₆, alcényle en C₂-C₆ linéaires ou ramifiés, ou R₂₂ et R₂₃ forment ensemble un groupement alcane di-yle-(CH₂)m₃- tel que m₃ représente un entier d'une valeur allant de 3 à 5, ou R₂₂ et R₂₃ forment ensemble un radical aralcane di-yle: 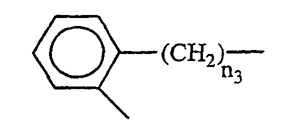 tel que n₃ représente 3 ou 4; ou un de leur sels.

De façon préférentielle, les acides de l'invention seront choisis parmi:
a) les composés répondant à la formule (I) dans laquelle R₅=R₆=H, p₁=q₁ et R₁=R'₁;
b) les composés répondant à la formule (II) dans laquelle r₂=1, R₁₃ =H, R₁₅=-COOH et m=1 (dérivés de la cystéine) ou m=2 (dérivés de l'homocystéine);
c) les composés répondant à la formule (III) dans laquelle R₂₂ est un radical aromatique, R₂₃=H et R₂₁=R'₂₁.

En particulier, les acides de l'invention sont choisis parmi: l'acide méthylène dithio-2,2' dibenzoïque, l'acide éthylène dithio-2,2' dibenzoïque, l'acide éthylène disulfinyl-2,2' dibenzoïque, l'acide (propane di-yl-1,3 dithio)-2,2' dibenzoïque, l'acide (butane di-yl-1,4 dithio)-2,2' dibenzoïque, l'acide (pentane di-yl-1,5 dithio)-2,2' dibenzoïque, l'acide (hexane di-yl-1,6 dithio)-2,2' dibenzoïque, l'acide (hexane di-yl-1,6 disulfinyl)-2,2' dibenzoïque, l'acide (hexane di-yl-1,6 dithio)-α,α' dibenzoïque, l'acide (octane di-yl-1,8 dithio)-2,2' dibenzoïque, l'acide (decane di-yl-1,10 dithio)-2,2' dibenzoïque, l'acide (decane di-yl-1,10 dithio)-α,α' dibenzoïque, l'acide méthylène dithio-α,α' diacétique, l'acide éthylène dithio-α,α' diacétique, l'acide éthylène disulfinyl -α,α' diacétique, l'acide éthylène disulfonyl -α,α' diacétique, l'acide (hexane di-yl-1,6 dithio)-α,α' diacétique, l'acide (hexane di-yl-1,6 disulfinyl)-α,α' diacétique, l'acide (hexane di-yl-1,6 disulfonyl)-α,α' diacétique, l'acide (decane di-yl-1,10 dithio)-α,α' diacétique, l'acide (decane di-yl-1,10 disulfinyl)-α,α' diacétique, l'acide (decane di-yl-1,10 disulfonyl)-α,α' diacétique, la S-(carboxyméthyl)-cystéine, la S-(carboxyéthyl)-cystéine, la S-(carboxy-4 butyl)-cystéine, la S-(carboxyméthyl)-sulfinyl cystéine, la S-(carboxyméthyl)-sulfonyl cystéine, la S-(carboxy-1 éthyl-1)-cystéine, la S-(carboxy-2 propyl-2)-cystéine, l'acide (p-méthoxy benzylidène dithio)-α,α' disuccinique, l'acide (benzylidène dithio)-α,α' disuccinique, l'acide (pipéronylidène dithio)-α,α' disuccinique, l'acide (vanillylidène dithio)-α,α' disuccinique, l'acide (vératrylidène dithio)-α,α' disuccinique, l'acide (butoxy-4 méthoxy-3 benzylidène dithio)-α,α' disuccinique, l'acide (carboxyméthyloxy-4 méthoxy-3 benzylidène dithio)-α,α' disuccinique, l'acide (p-méthoxy benzylidène dithio)-α,α' diacétique, l'acide (benzylidène dithio)-α,α' diacétique, l'acide (pipéronylidène dithio)-α,α' diacétique, l'acide (vanillylidène dithio)-α,α' diacétique, l'acide (vératrylidène dithio)-α,α' diacétique, ainsi que leurs mono- et leurs di-sels de base minérale ou organique.

De préférence on utilisera selon l'invention la S-carboxyméthylcystéine ainsi que ses mono- et ses di-sels de base minérale ou organique.

Ces composés contenant une fonction thio-éther, sulfoxide ou sulfone sont utilisés comme agents cosmétiques piégeurs de gaz toxiques.

La présente invention a également pour objet l'utilisation de composés contenant une fonction thio-éther, sulfoxide ou sulfone, dans ou pour la préparation d'une composition cosmétique à application topique anti-polluante.

Les compositions cosmétiques utilisées dans l'invention contiendront avantageusement de 0,001% à 10%, de préférence entre 0,01 et 5% en poids de composés contenant une fonction thio-éther, sulfoxide ou sulfone par rapport au poids total de la composition.

Cette composition peut contenir en outre au moins un autre composé anti-pollution.

Celui-ci peut notamment être choisi parmi les anthocyanes et/ou ses dérivés, l'ergothionine et/ou ses dérivés, les chélateurs de métaux comme les dérivés de l'acide N,N'-dibenzyl éthylène diamine N,N'-diacétique, les antioxydants, les extraits cellulaires de végétal de la famille Pontederiaceae. Parmis les antioxydants, on choisira plus paticulièrement des polyphénols comme l'acide ellagique.

La composition cosmétique utilisée dans l'invention peut contenir en outre un milieu cosmétiquement acceptable, qui est plus particulièrement constitué d'eau et/ou de solvant organique cosmétiquement acceptable.

Ils peuvent être choisis dans le groupe constitué par les solvants organiques hydrophiles, les solvants amphiphiles, les solvants organiques lipophiles ou leurs mélanges.

Parmi les solvants organiques hydrophiles, on peut citer par exemple des mono-alcools inférieurs, linéaires ou ramifiés, ayant de 1 à 8 atomes de carbone comme l'éthanol, le propanol, le butanol, l'isopropanol, l'isobutanol, les polyéthylèneglycols ayant de 6 à 80 oxydes d'éthylène, les polyols tels que le propylèneglycol, l'isoprène glycol, le butylèneglycol, le glycérol, le sorbitol, les mono- ou dialkyles d'isosorbide dont les groupements alkyle ont de 1 à 5 atomes de carbone comme le diméthyl isosorbide, les éthers de glycol comme le diéthylène glycol mono-méthyle ou mono-éthyléther et les éthers de propylène glycol comme le dipropylène glycol méthyléther.

Comme solvants organiques amphiphiles, on peut citer des polyols tels que des dérivés de propylèneglycol (PPG), tels que les esters de polypropylèneglycol et d'acides gras, de PPG et d'alcools gras comme le PPG-23 oleyléther et le PPG-36 oléate.

Comme solvants organiques lipophiles, on peut citer par exemple les esters gras tels que l'adipate de diisopropyle, l'adipate de dioctyle, les benzoates d'alkyle.

Les solvants organiques sont choisis préférentiellement parmi les alcools mono- ou polyfonctionnels, les polyéthylène glycols éventuellement oxyéthylénés, les esters de polypropylène glycol, le sorbitol et ses dérivés, les dialkyls d'isosorbide, les éthers de glycol et des éthers de polypropylène glycol, les esters gras.

Les solvants organiques peuvent représenter de 5 à 98% du poids total de la composition.

Afin que les compositions utilisées dans l'invention soient plus agréables à utiliser, plus douces à l'application, plus nourrissantes et plus émolluantes, il est possible d'ajouter une phase grasse dans le milieu de ces compositions.

La phase grasse représente, de préférence, de 0 à 50% en poids total de la composition.

Cette phase grasse peut comporter une ou plusieurs huiles choisies de préférence dans le groupe constitué par :
- les silicones volatiles ou non-volatiles, linéaires, ramifiées ou cycliques, organo-modifiées ou non, hydrosolubles ou liposolubles,
- les huiles minérales telles que l'huile de paraffine et de vaseline,
- les huiles d'origine animale telles que le perhydrosqualène,
- les huiles d'origine végétale telles que l'huile d'amandes douces, l'huile d'avocat, l'huile de ricin, l'huile d'olive, l'huile de jojoba, l'huile de sésame, l'huile d'arachide, l'huile de macadamia, l'huile de pépins de raisin, l'huile de colza, l'huile de coprah,
- les huiles synthétiques telles que l'huile de Purcellin, les isoparaffines,
- les huiles fluorées et perfluorées,
- les esters d'acides gras tels que l'huile de Purcellin.

Elle peut aussi comporter comme matières grasses (un) ou plusieurs alcools gras, acides gras ou cires (paraffine, cire de polyéthylène, Carnauba, cire d'abeilles).

De façon connue, les compositions utilisées dans l'invention peuvent en outre contenir des adjuvants habituels dans le domaine cosmétique les gélifiants et/ou épaississants classiques aqueux ou lipophiles, les actifs hydrophiles ou lipophiles, les conservateurs, des antioxydants, les parfums, les émulsionnants, les agents hydratants, les agents pigmentants, les dépigmentants, les agents kératolytiques, les vitamines, les émollients, les séquestrants, les tensio-actifs, les polymères, les agents alcalinisants ou acidifiants, les charges, les agents anti-radicaux libres, les céramides, les filtres solaires, notamment ultra-violets, les répulsifs pour insectes, les agents amincissants, les matières colorantes, les bactéricides, les anti-pelliculaires.

Les quantités de ces différents adjuvants sont celles classiquement utilisées dans les domaines considérés.

Bien entendu, l'homme de l'art veillera à choisir le ou les éventuels composés à ajouter à la composition selon l'invention, de manière telle que les propriétés avantageuses attachées intrinsèquement à la composition conforme à l'invention ne soient pas, ou substantiellement pas, altérées par l'addition envisagée.

Les compositions utilisées selon l'invention peuvent se présenter sous toutes les formes galléniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide ou d'une dispersion d'huile dans une phase aqueuse à l'aide de sphérules, ces sphérules pouvant être des nanoparticules polymériques telles que les nanosphères et les nanocapsules, ou mieux, des vésicules lipidiques de type ionique et/ou non-ionique.

Les compositions utilisées dans la présente invention peuvent être plus ou moins fluides et avoir l'aspect d'une crème blanche ou colorée, d'une pommade, d'un lait, d'une lotion, d'un sérum, d'une pâte, d'une mousse ou d'un solide.

Elles peuvent éventuellement être appliquées sur la peau sous forme d'aérosol.

Elles peuvent également se présenter sous forme solide, et par exemple sous forme de stick.

Elles peuvent être utilisées comme produit de soin et/ou comme produit de maquillage.

Un autre objet de l'invention consiste en un procédé de traitement cosmétique destiné à obtenir une protection de l'organisme contre les effets de la pollution, consistant à appliquer sur la peau une quantité cosmétiquement efficace de composés contenant une fonction thio-éther, sulfoxide ou sulfone.

Un autre procédé de traitement cosmétique selon l'invention destiné à obtenir une protection de l'organisme contre les effets de la pollution, consiste à appliquer sur la peau une composition cosmétique selon l'invention, telle que définie ci-dessus.

Les exemples qui suivent sont destinés à illustrer l'invention sans pour autant présenter un caractère limitatif.

### EXPERIMENTATION

### Principe :

L'ozone a la capacité d'oxyder les constituants cellulaires en générant notamment des protéines carbonylées et des hydropéroxydes lipidiques. La quantification des hydropéroxydes lipidiques est un moyen de mesure du stress oxydatif induit par une exposition du tissu cutané à ce polluant. Une diminution de leur taux indique un effet protecteur.

### Type cellulaire et culture :

L'étude a été réalisée sur une culture monocouche de kératinocytes humains. Les cellules sont ensemencées à J-3 en boîtes 48 puits à raison de 25000 cellules/cm² dans 500 µl de milieu de culture. Les incubations sont réalisées à 37°C, 5% de CO₂ en atmosphère humide.

### Pré-traitement des kératinocytes par la S-carboxyméthylcystéine (CMC):

Les cellules ont été prétraitées 24 heures avec la CMC (0,5 mg/ml, demi-dose maximale non toxique pour les cellules, solubilisée dans le milieu de culture).

### Incorporation d'un marqueur de stress oxydant, DCFH-DA (2,7-dichlorofluorescindiacétate) :

Les hydropéroxydes constituent un marqueur du stress intracellulaire. Ils sont détectés et quantifiés au moyen d'une technique fluorescente (Lebel C.P., Ischiropoulos H. and Bondy S.C. (1992) Evaluation of the probe 2,7-dichlorofluorescin as an indicator of Reactive Oxygen Species formation and oxydative stress. Chem. Res. Toxicol.; 5: 227-231).

En présence d'hydropéroxydes et des péroxydases intracellulaires, la DCFH est oxydée en 2, 7-dichlorofluorescéine (DCF) fluorescente.

Les cellules, prétraitées 24 heures avec la CMC, sont lavées par du tampon PBS et mises en contact 30 minutes avec une solution de DCFH-DA (500 µl/puits), préparée dans le milieu de culture à la concentration de 320 µM.

### Exposition à l'ozone :

Les cellules sont de nouveau rincées avec du tampon PBS puis mises en contact avec une solution à 1mg/ml de CMC dans le PBS (100µl/puits). Elles sont ensuite exposées à l'ozone (1 ppm), en atmosphère humide, dans un incubateur régulé à 37°C.

### Mesures des hydropéroxydes lipidiques induits par l'ozone :

La formation de DCF fluorescente (filtres d'excitation à 485 nm et d'émission à 530 nm) résultant de la production d'hydropéroxydes, est mesurée après différents temps d'exposition à l'ozone : 0, 5, 10 et 20 minute.

### Résultats:

Toxicité de l'ozone envers les kératinoctes humains en culture, en absence et en présence de S-carboxyméthylcystéine à la concentration de 200 µM., en fonction du temps d'exposition (n=4).

| **Fluorescence observée en présence de CMC, exprimée en % par rapport aux témoins non protégés pour chaque temps** | | |
|---|---|---|
| 5 min. de contact | 10 min. de contact | 20 min. de contact |
| % fluorescence observée +/- SEM | % fluorescence observée +/- SEM | % fluorescence observée +/- SEM |
| **32,4 +/- 5,9** | **33,7 +/- 5,0** | **46,5 +/- 8,5** |

Pour chaque temps, les valeurs de fluorescence des témoins non protégés sont ramenées à 100%. Les résultats, en présence de CMC sont alors exprimés par rapport à cette valeur témoin. La CMC diminue de façon significative le stress induit par l'ozone. Cette protection est maximale dès 5 minutes d'exposition (chute de 67,7% du stress induit). Elle est toujours significative après 20 minutes d'exposition (chute de 53,5% du stress induit) par rapport aux témoins non protégés.

A partir d'un modèle biologique, in vitro, utilisant une cellule d'origine épithéliale humaine (kératinocyte/peau), nous avons montré :
- qu'un agent représentatif d'une catégorie de polluants atmosphériques comme l'ozone, entraîne dans nos conditions expérimentales l'apparition d'un stress oxydant important,
- que la CMC exerce un effet protecteur hautement significatif contre l'effet induit par ce polluant.

### EXEMPLES DE FORMULATION

**Exemple 1:** Selon les techniques usuelles de préparation, on mélange les constituants ci-dessous pour préparer une émulsion.

### COMPOSITION POUR L'APPLICATION TOPIQUE

| | |
|---|---|
| S-carboxyméthylcystéine | 5 g |
| polyéthylèneglycol oxyéthyléné par 50 moles d'oxyde d'éthylène | 3 g |
| monodiglycérylstéarate | 3 g |
| huile de vaseline | 24 g |
| alcool cétylique | 5 g |
| eau qsp | 100 g |

**Exemple 2:** De la même manière, on prépare une émulsion selon une technique classique, à partir des composés suivants:

| | |
|---|---|
| S-carboxyméthylcystéine | 1 g |
| octylpalmitate | 10 g |
| glycérylisostéarate | 4 g |
| huile de purcellin | 23 g |
| vitamine E | 1 g |
| glycérol | 3 g |
| eau qsp | 100 g |

**Exemple 3:** De la même manière, on prépare une émulsion selon une technique classique, à partir des composés suivants:

| | |
|---|---|
| Acide (hexane di-yl-1,6-dithio)α,α' diacétique | 1 g |
| octylpalmitate | 10 g |
| glycérylisostéarate | 4 g |
| huile de vaseline | 20 g |
| sorbitol | 2 g |
| vitamine E | 1 g |
| glycérol | 3 g |
| eau qsp | 100 g |

**Exemple 4:** De la même manière, on prépare une émulsion selon une technique classique, à partir des composés suivants:

| | |
|---|---|
| Acide (décane di-yl-1,10 disulfinyl)-α,α' diacétique | 1 g |
| octylpalmitate | 10 g |
| glycérylisostéarate | 4 g |
| huile de vaseline | 24 g |
| vitamine E | 1 g |
| glycérol | 3 g |
| eau qsp | 100 g |

**Exemple 5:** A partir des constituants ci-dessous, on formule la composition suivante:

| | |
|---|---|
| Acide (p-méthoxybenzylidène dithio)-α,α' disuccinique | 1,5 g |
| huile de jojoba | 13 g |
| parabenzoxy benzoate de méthyle et d'isopropyle | 0,05 g |
| sorbate de potassium | 0,3 g |
| cyclopentadimethylsiloxane | 10 g |
| alcool stéarylique | 1 g |
| acide stéarique | 4 g |
| stéarate de polyéthylèneglycol | 3 g |
| vitamine E | 1 g |
| glycérol | 3 g |
| eau qsp | 100 g |

**Exemple 6:** A partir des constituants ci-dessous, on formule la composition suivante:

| | |
|---|---|
| Acide (p-méthoxybenzylidène dithio)-α,α' diacétique | 1 g |
| huile de jojoba | 13 g |
| parabenzoxy benzoate de méthyle et d'isopropyle | 0,05 g |
| sorbate de potassium | 0,3 g |
| cyclopentadimethylsiloxane | 10 g |
| alcool stéarylique | 1 g |
| acide N,N'-di-(3-hydroxybenzyle)-éthylène diamine N,N'-diacétique | 0,01 g |
| acide stéarique | 4 g |
| stéarate de polyéthylèneglycol | 3 g |
| vitamine E | 1 g |
| glycérol | 3 g |
| eau qsp | 100 g |

**Exemple 7:** A partir des constituants ci-dessous, on formule la composition suivante:

| | |
|---|---|
| Acide (vanillylidène dithio)-α,α' diacétique | 0,5 g |
| huile de jojoba | 13 g |
| parabenzoxy benzoate de méthyle et d'isopropyle | 0,05 g |
| sorbate de potassium | 0,3 g |
| cyclopentadimethylsiloxane | 10 g |
| alcool stéarylique | 1 g |
| acide stéarique | 4 g |
| extrait cellulaires de jacinthe d'eau | 0,05 g |
| stéarate de polyéthylèneglycol | 3 g |
| vitamine E | 1 g |
| glycérol | 3 g |
| eau qsp | 100 g |

L'extrait cellulaires de jacinthe d'eau de la famille Pontederiaceae (*Eichhornia crassipes*) a été obtenu par ce procédé: 12 pieds de jacinthe d'eau ont été lavés à l'eau puis grossièrement essorés. Passés en broyeur à couteau (robot coupe), on a obtenu 700 g de broyat. Ajout de 700 ml d'H₂O, puis 300 ml d'H₂O MilliQ. Nouveau passage en robot coupe 5 minutes, centrifugation 20'/8000 G filtration Whatmann GFD puis GFF et lyophilisation: 5,43 g de lyophilisat sont ainsi obtenus.

## Revendications

1. Utilisation pour la préparation d'un agent topique de protection de la peau et des matières kératiniques de façon à prévenir, atténuer ou supprimer les effets délétères des gaz toxiques et des composés organiques résidus de combustions, d'au moins un dérivé à fonction thio-éther, sulfoxide ou sulfone de formule (I), (II) ou (III): 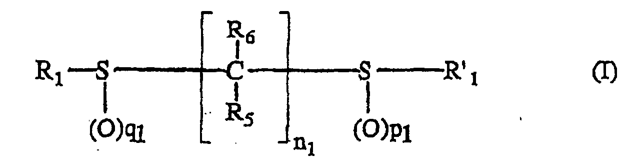 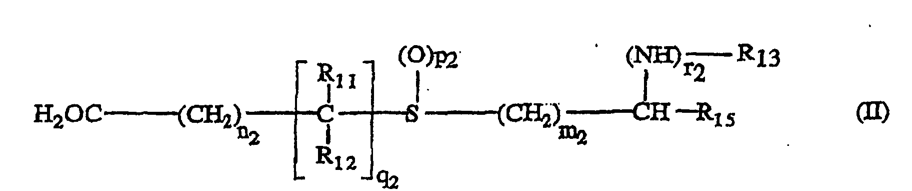 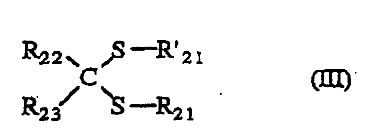 dans lesquelles n₁ représente un nombre entier d'une valeur allant de 1 à 10, p₁ et q₁, identiques ou différents, représentent 0, 1 ou 2, n₂ représente un nombre entier d'une valeur allant de 0 à 5, p₂ représente 0, 1 ou 2, q₂ représente 0, 1, 2 ou 3, m₂ représente un nombre entier d'une valeur allant de 0 à 10, r₂ représente 0 ou 1, q₂ et n₂ n'étant jamais tous deux nuls simultanément, R₁ et R'₁, identiques ou différents, représentent un radical choisi parmi les groupements:
a) 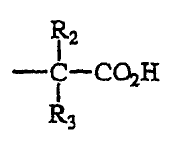
b) 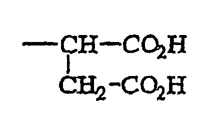
c) 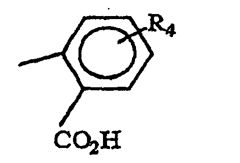
dans lesquels, R₂ et R₃, identiques ou différents désignent un atome d'hydrogène ou un groupement alcoyle en C₁-C₈ ou alcényle C₂-C₈, linéaires ou ramifiés; R₄ désigne un atome d'hydrogène ou un groupement alcoyle en C₁-C₈ linéaire ou ramifie, un groupement alcényle en C₂-C₈ linéaire ou ramifié, un groupement alcoxy en C₁-C₈ linéaire ou ramifié; R₅ et R₆ désignent indépendamment l'un de l'autre un atome d'hydrogène, alcényle en C₂-C₈, alcoxy en C₁-C₈ linéaire ou ramifié, phényle, alcoylcarboxylique en C₁-C₆, alcénylcarboxylique en C₂-C₆; R₁₁ et R₁₂ ont les mêmes significations que R₅ et R₆; R₁₃ représente un groupement alcoyle en C₁-C₁₇ ou alcényle en C₂-C₁₇ linéaires ou ramifiés ou un radical acyle-CO-R₁₄, avec R₁₄ qui désigne un groupement alcoyle en C₁-C₁₇ ou alcényle en C₂-C₁₇ linéaires ou ramifiés; R₁₅ est choisi parmi un atome d'hydrogène, un groupement alcoyle en C₁-C₈, alcényle en C₂-C₈ linéaires ou ramifiés, un groupement alcoylcarboxylique en C₁-C₅, alcénylcarboxylique en C₂-C₅, ou la fonction -COOH; R₂₁ et R'₂₁ désignent les groupements de formule : 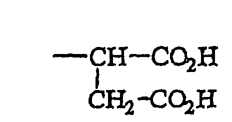 et
―CH₂-CO₂H
R₂₂ représente un groupement :
i)
―CH₃
ii)
―CO₂H
iii) 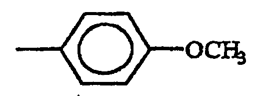
iv) 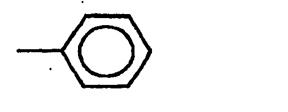
v) 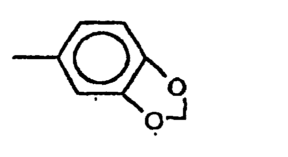
vi) 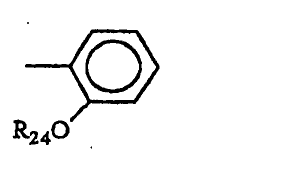
vii) 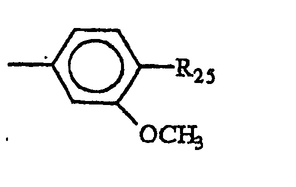
dans lesquels R₂₄ représente un groupement choisi parmi: -CH₂-COOH et -CO-CH₃ et R₂₅ représente un groupement choisi parmi: OH, OCH₃, OC(CH₃)₃, OCH₂COOH;
R₂₃ désigne un atome d'hydrogène, un radical alcoyle en C₁-C₆, alcényle en C₂-C₆ linéaires ou ramifiés, ou R₂₂ et R₂₃ forment ensemble un groupement alcane di-yle-(CH₂)m₃- tel que m₃ représente un entier d'une valeur allant de 3 à 5, ou R₂₂ et R₂₃ forment ensemble un radical aralcane di-yle 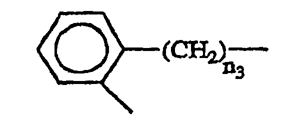 tel que n₃ représente 3 ou 4; ou un de leur sels.

2. Utilisation cosmétique d'un dérivé à fonction thio-éther, sulfoxide ou sulfone de formule (I), (II) ou (III) tel que défini à la revendication 1, en application topique, comme agent de protection de la peau et des matières kératiniques de façon à prévenir, atténuer ou supprimer les effets délétères des gaz toxiques et des composés organiques résidus de combustions.

3. Utilisation selon la revendication 1 ou 2, **caractérisée en ce que** le dérivé à fonction thio-éther répond à la formule (I) dans laquelle R₅=R₆=H, p₁=q₁ et R₁=R'_{1.}

4. Utilisation selon la revendication 1 ou 2, **caractérisée en ce que** le dérivé à fonction sulfoxide répond à la formule (II) dans laquelle r₂=1, R₁₃=H, R₁₅=-COOH et m=1 (dérivés de la cystéine) ou m=2 (dérivés de l'homocystéine).

5. Utilisation selon la revendication 1 ou 2, **caractérisée en ce que** le dérivé à fonction sulfone répond à la formule (III) dans laquelle R₂₂ est un radical aromatique, R₂₃=H et R₂₁=R'₂₁.

6. Utilisation selon l'une quelconque des revendications 1 à 5, **caractérisée en ce que** le dérivé à fonction thio-éther, sulfoxide ou sulfone est choisi parmi l'acide méthylène dithio-2,2' dibenzoïque, l'acide éthylène dithio-2,2' dibenzoïque, l'acide éthylène disulfinyl-2,2' dibenzoïque, l'acide (propane di-yl-1,3 dithio)-2,2' dibenzoïque, l'acide (butane di-yl-1,4 dithio)-2,2' dibenzoïque, l'acide (pentane di-yl-1,5 dithio)-2,2' dibenzoïque, l'acide (hexane di-yl-1,6 dithio)-2,2' dibenzoïque, l'acide (hexane di-yl-1,6 disulfinyl)-2,2' dibenzoïque, l'acide (hexane di-yl-1,6 dithio)-α,α' dibenzoïque, l'acide (octane di-yl-1,8 dithio)-2,2' dibenzoïque, l'acide (decane di-yl-1,10 dithio)-2,2' dibenzoïque, l'acide (decane di-yl-1,10 dithio)-α,α' dibenzoïque, l'acide méthylène dithio-α,α' diacétique, l'acide éthylène dithio-α,α' diacétique, l'acide éthylène disulfinyl -α,α' diacétique, l'acide éthylène disulfonyl-α,α' diacétique, l'acide (hexane di-yl-1,6 dithio)-α,α' diacétique, l'acide (hexane di-yl-1,6 disulfinyl)-α,α' diacétique, l'acide (hexane di-yl-1,6 disulfonyl)-α,α' diacétique, l'acide (decane di-yl-1,10 dithio)-α,α' diacétique, l'acide (decane di-yl-1,10 disulfinyl)-α,α' diacétique, l'acide (decane di-yl-1,10 disulfonyl)-α,α' diacétique, la S-(carboxyméthyl)-cystéine, la S-(carboxyéthyl)-cystéine, la S-(carboxy-4 butyl)-cystéine, la S-(carboxyméthyl)-sulfinyl cystéine, la S-(carboxyméthyl)-sulfonyl cystéine, la S-(carboxy-1 éthyl-1)-cystéine, la S-(carboxy-2 propyl-2)-cystéine, l'acide (p-méthoxy benzylidène dithio)-α,α' disuccinique, l'acide (benzylidène dithio)-α,α' disuccinique, l'acide (pipéronylidène dithio)-α,α' disuccinique, l'acide (vanillylidène dithio)-α,α' disuccinique, l'acide (vératrylidène dithio)-α,α' disuccinique, l'acide (butoxy-4 méthoxy-3 benzylidène dithio)-α,α' disuccinique, l'acide (carboxyméthyloxy-4 méthoxy-3 benzylidène dithio)-α,α' disuccinique, l'acide (p-méthoxy benzylidène dithio)-α,α' diacétique, l'acide (benzylidène dithio)-α,α' diacétique, l'acide (pipéronylidène dithio)-α,α' diacétique, l'acide (vanillylidène dithio)-α,α' diacétique, l'acide (vératrylidène dithio)-α,α' diacétique, ainsi que leurs mono- et leurs di-sels de base minérale ou organique.

7. Utilisation selon l'une quelconque des revendications 1 à 6, **caractérisée en ce que** le dérivé à fonction thio-éther, sulfoxide ou sulfone de formule (I), (II) ou (III) est choisi parmi la carboxyméthylcystéine ainsi que ses mono- et ses di-sels de base minérale ou organique.

8. Utilisation en application topique d'au moins un dérivé à fonction thio-éther, sulfoxide ou sulfone de formule (I), (II) ou (III) telle que définie dans l'une des revendications 2 à 7, comme agent cosmétique piégeur de gaz toxiques tels que l'ozone.

9. Utilisation d'un dérivé à fonction thio-éther, sulfoxide ou sulfone de formule (I), (II) ou (III) telle que défini selon l'une quelconque des revendications 1 ou 3 à 7 dans la préparation d'une composition, topique de protection de la peau et des matières kératiniques de façon à prévenir, atténuer ou suppimer les effets délétères des gaz toxiques et des composés organiques résidus de combustions.

10. Utilisation cosmétique selon l'une des revendications 2 à 7, **caractérisée en ce que** le dérivé à fonction thio-éther, sulfoxide ou sulfone de formule (I), (II) ou (III) est utilisé dans une composition cosmétique à application topique de protection de la peau et des matières kératiniques de facon à prévenir, atténuer ou supprimer les effets délétères des gaz toxiques et des composés organiques résidus de combustion.

11. Utilisation selon la revendication 10, **caractérisée en ce que** ladite composition cosmétique contient de 0,005% à 10% et de préférence de 0,1 à 5% en poids de composé à fonction thio-éther, sulfoxide ou sulfone de formule (I), (II) ou (III) par rapport au poids total de la composition.

12. Utilisation selon la revendication 10 ou 11, **caractérisée en ce que** ladite composition contient en outre au moins un autre composé anti-pollution.

13. Utilisation selon la revendication 12, **caractérisée en ce que** ledit composé anti-pollution est choisi parmi les anthocyanes et/ou ses dérivés, l'ergothionine et/ou ses dérivés, les chélateurs de métaux comme les dérivés de l'acide N,N'-dibenzyl éthylène diamine N,N'-diacétique, les antioxydants comme l'acide ellagique, les extraits cellulaires de végétal de la famille Pontederiaceae.

14. Utilisation selon l'une quelconque des revendications 10 a 13, **caractérisée en ce que** la composition contient en outre un milieu cosmétiquement acceptable constitué d'eau et/ou d'au moins un solvant organique choisi dans le groupe constitué par les solvants organiques hydrophiles, les solvants organiques lipophiles, les solvants amphiphiles ou leurs mélanges.

15. Utilisation selon la revendication 14, **caractérisée en ce que** les solvants organiques sont choisis dans le groupe constitué par les alcools mono- ou polyfonctionnels, les polyéthylène glycols éventuellement oxyéthylénés, les esters de polypropylène glycol, le sorbitol et ses dérivés, les dialkyls d'isosorbide, les éthers de glycol et des éthers de polypropylène glycol, les esters gras.

16. Utilisation selon la revendication 14 ou 15, caractérisée on ce que le ou les solvants organiques représentent de 5 à 98% du poids total de la composition.

17. Utilisation selon l'une quelconque des revendications 10 à 16, **caractérisée en ce que** la composition comprend en outre au moins une phase grasse.

18. Utilisation selon la revendication 17, **caractérisée en ce que** la phase grasse représente de 0 à 50% du poids total de la composition.

19. Utilisation selon l'une quelconque des revendications 10 à 18, **caractérisée par le fait que** la composition contient en outre au moins un additif choisi dans le groupe constitué par les gélifiants et/ou épaississants classiques aqueux ou lipophiles, les actifs hydrophiles ou lipophiles, les conservateurs, des antioxydants, les parfums, les émulsionnants, les agents hydratants, les agents pigmentants, les dépigmentants, les agents kératolytiques, les vitamines, les émollients, les séquestrants, les tensio-actifs, les polymères, les agents slcalinisants ou acidifiants, les charges, les agents anti-radicaux libres, les céramides, les filtres solaires, notamment ultra-violets, les répulsifs pour insectes, les agents amincissants, les matières colorantes, les bactéricides, les anti-pelliculaires.

20. Utilisation selon l'une quelconque des revendications 10 à 19, **caractérisée en ce que** la composition se présente sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide ou d'une dispersion d'huile dans une phase aqueuse à l'aide de sphérules.

21. Utilisation selon l'une quelconque des revendications 10 à 20, **caractérisée en ce que** la composition a l'aspect d'une crème blanche ou colorée, d'une pommade, d'un lait, d'une lotion, d'un sérum, d'une pâte, d'une mousse ou d'un solide.

22. Utilisation selon l'une quelconque des revendications 10 à 21, **caractérisée en ce que** la composition présente un pH compris entre 3 et 8.

23. Procédé cosmétique de protection de l'organisme contre les effets de la pollution, **caractérisé en ce qu'**il consiste à appliquer sur la matière kératinique une quantité cosmétiquement efficace d'au moins un dérivé à fonction thio-éther, sulfoxide ou sulfone de formule (I), (II) ou (III) tel que défini dans l'une des revendications 1 à ou 3 à 7.

## Patentansprüche

1. Verwendung mindestens eines Derivats mit Thioetherfunktion, Sulfoxidfunktion oder Sulfonfunktion der Formel (I), (II) oder (III) zur Herstellung eines topisch angewandten Mittels zum Schutz der Haut und der Keratinsubstanzen zur Vorbeugung, Abschwächung oder Unterdrückung der schädlichen Wirkungen von toxischen Gasen und organischen Verbrennungsrückständen: 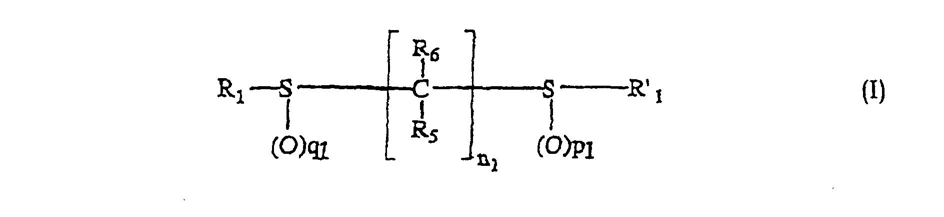 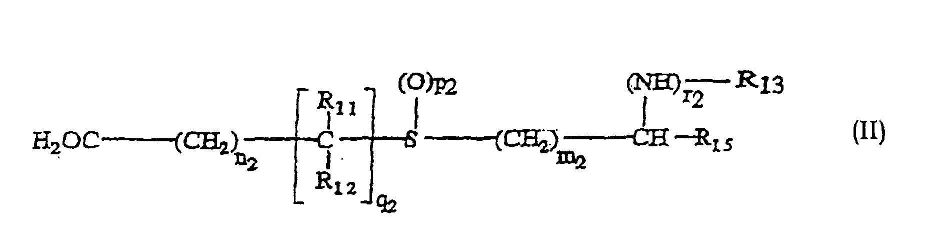 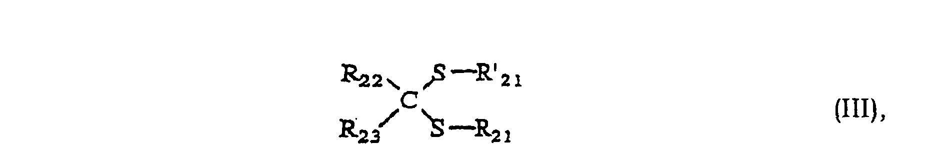 worin n₁ eine ganze Zahl im Bereich von 1 bis 10 ist, p₁ und q₁, die gleich oder verschieden sind, 0, 1 oder 2 bedeuten, n₂ 0 bedeutet oder eine ganze Zahl im Bereich von 1 bis 5, p₂ 0,1 oder 2 bedeutet, q₂ 0,1, 2 oder 3 bedeutet, m₂ 0 ist oder eine ganze Zahl von 1 bis 10 bedeutet und r₂ 0 oder 1 bedeutet, wobei q₂ und n₂ nicht gleichzeitig beide 0 sind, und R₁ und R'₁, die gleich oder verschieden sind, eine Gruppe bedeuten, die unter den folgenden Gruppen ausgewählt ist:
a) 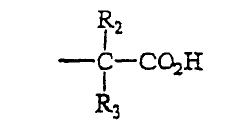
b) 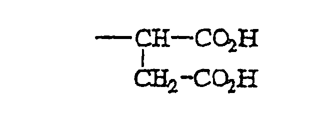
c) 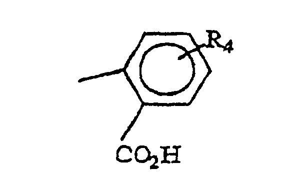
worin bedeuten: R₂ und R₃, die gleich oder verschieden sind, ein Wasserstoffatom, eine geradkettige oder verzweigte C₁₋₈-Alkylgruppe oder eine geradkettige oder verzweigte C₂₋₈-Alkenylgruppe; R₄ ein Wasserstoffatom, eine geradkettige oder verzweigte C₁₋₈-Alkylgruppe, eine geradkettige oder verzweigte C₂₋₈-Alkenylgruppe oder eine geradkettige oder verzweigte C₁₋₈-Alkoxygruppe;
R₅ und R₆ unabhängig voneinander ein Wasserstoffatom, eine C₂₋₈-Alkenylgruppe, eine geradkettige oder verzweigte C₁₋₈-Alkoxygruppe, eine Phenylgruppe, eine C₁₋₆-Alkylcarboxygruppe oder eine C₂₋₆-Alkenylcarboxygruppe; R₁₁ und R₁₂ die für R₅ und R₆ angegebenen Bedeutungen; R₁₃ eine geradkettige oder verzweigte C₁₋₁₇-Alkylgruppe, eine geradkettige oder verzweigte C₂₋₁₇-Alkenylgruppe oder eine Alkyl-CO-R₁₄-Gruppe, wobei R₁₄ eine geradkettige oder verzweigte C₁₋₁₇-Alkylgruppe oder eine geradkettige oder verzweigte C₂₋₁₇-Alkenylgruppe ist; R₁₅ ein Wasserstoffatom, eine geradkettige oder verzweigte C₁₋₈-Alkylgruppe, eine geradkettige oder verzweigte C₂₋₈-Alkenylgruppe, eine C₁₋₅-Alkylcarboxygruppe, eine C₂₋₅-Alkenylcarboxygruppe oder die Gruppe -COOH; R₂₁ und R'₂₁ Gruppen der folgenden Formel: 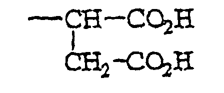 und
―CH₂-CO₂H ;
R₂₂ eine Gruppe:
i)
―CH₃
ii)
―CO₂H
iii) 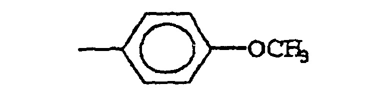
iv) 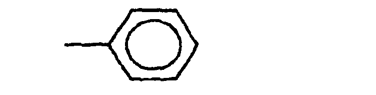
v) 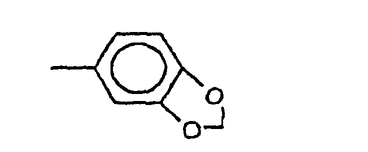
vi) 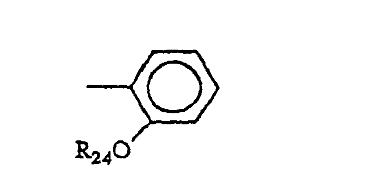
vii) 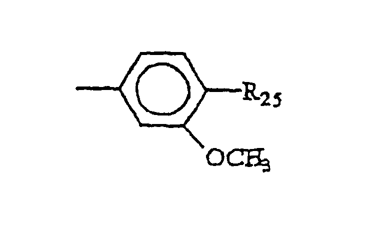
wobei R₂₄ eine Gruppe bedeutet, die ausgewählt ist unter: -CH₂-COOH und -CO-CH₃; und R₂₅ eine Gruppe bedeutet, die ausgewählt ist unter: OH, OCH₃, OC(CH₃)₃, OCH₂COOH;
R₂₃ ein Wasserstoffatom, eine geradkettige oder verzweigte C₁₋₆-Alkylgruppe oder eine geradkettige oder verzweigte C₂₋₆-Alkenylgruppe oder R₂₂ und R₂₃ bilden gemeinsam eine Alkandiylgruppe (CH₂)ₘ₃, wobei m₃ eine ganze Zahl von 3 bis 5 ist, oder R₂₂ und R₂₃ bilden gemeinsam eine Aralkandiylgruppe 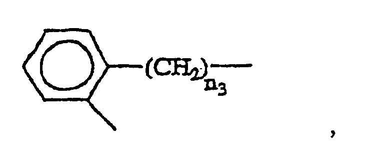 wobei n₃ 3 oder 4 bedeutet; oder eines Salzes dieser Verbindungen.

2. Kosmetische Verwendung eines Derivats mit Thioetherfunktion, Sulfoxidfunktion oder Sulfonfunktion der Formel (I), (II) oder (III) nach Anspruch 1 zur topischen Anwendung als Mittel zum Schutz der Haut und der Keratinsubstanzen, um den schädlichen Wirkungen von toxischen Gasen und organischen Verbrennungsrückständen vorzubeugen, sie abzuschwächen oder sie zu unterdrücken.

3. Verwendung nach Anspruch 1 oder 2, **dadurch gekennzeichnet, dass** das Derivat mit Thioetherfunktion der Formel (I) entspricht, wobei R₅=R₆=H, p₁=q₁ und R₁=R'₁.

4. Verwendung nach Anspruch 1 oder 2, **dadurch gekennzeichnet, dass** das Derivat mit Sulfoxidfunktion der Formel (II) entspricht, wobei r₂=1, R₁₃=H, R₁₅₌-COOH und m=1 (Cysteinderivate) oder m=2 (Homocysteinderivate).

5. Verwendung nach Anspruch 1 oder 2, **dadurch gekennzeichnet, dass** das Derivat mit Sulfonfunktion der Formel (III) entspricht, wobei R₂₂ eine aromatische Gruppe bedeutet, R₂₃=H und R₂₁=R'₂₁.

6. Verwendung nach einem der Ansprüche 1 bis 5, **dadurch gekennzeichnet, dass** das Derivat mit Thioetherfunktion, Sulfoxidfunktion oder Sulfonfunktion unter 2,2'-Methylendithiodibenzoesäure, 2,2'-Ethylendithiodibenzoesäure, 2,2'-Ethylendisulfinyldibenzoesäure, 2,2'-(1,3-Propandiyldithio)-dibenzoesäure, 2,2'-(1,4-Butandiyldithio)-dibenzoesäure, 2,2'-(1,5-Pentandiyldithio)-dibenzoesäure, 2,2'-(1,6-Hexandiyldithio)-dibenzoesäure, 2,2'-(1,6-Hexandiyldisulfinyl)-dibenzoesäure, α,α'-(1,6-Hexandiyldithio)-dibenzoesäure, 2,2'-(1,8-Octandiyldithio)-dibenzoesäure, 2,2'-(1,10-Decandiyldithio)-dibenzoesäure, α,α'-(1,10-Decandiyldithio)-dibenzoesäure, α,α'-Methylendithiodiessigsäure, α,α'-Ethylendithio-diessigsäure, α,α'-Ethylendisulfinyl-diessigsäure, α,α'-Ethylendisulfonyl-diessigsäure, α,α'-(1,6-Hexandiyldithio)-diessigsäure, α,α'-(1,6-Hexandiyldisulfinyl)-diessigsäure, α,α'-(1,6-Hexandiyldisulfonyl)-diessigsäure, α,α'-(1,10-Decandiyldithio)-diessigsäure, α,α'-(1,10-Decandiyldisulfinyl)-diessigsäure, α,α'-(1,10-Decandiyldisulfonyl)-diessigsäure, S-(Carboxymethyl)-cystein, S-(Carboxyethyl)-cystein, S-(4-Carboxybutyl)-cystein, S-(Carboxymethyl)-sulfinyl-cystein, S-(Carboxymethyl)-sulfonyl-cystein, S-(1-Carboxy-1-ethyl)-cystein, S-(2-Carboxy-2-propyl)-cystein, α,α'-(p-Methoxybenzylidendithio)-dibernsteinsäure, α,α'-(Benzylidendithio)-dibemsteinsäure, α,α'-(Piperonylidendithio)-dibernsteinsäure, α,α'-(Vanillylidendithio)-dibernsteinsäure, α,α'-(Veratrylidendithio)-dibemsteinsäure, α,α'-(4-Butoxy-3-methoxybenzylidendithio)-dibernsteinsäure, α,α'-(4-Carboxymethyloxy-3-methoxybenzylidendithio)-dibernsteinsäure, α,α'-(p-Methoxybenzylidendithio)-diessigsäure, α,α'-(Benzylidendithio)-diessigsäure, α,α'-(Piperonylidendithio)-diessigsäure, α,α'-(Vanillylidendithio)-diessigsäure, α,α'-Veratrylidendithio)-diessigsäure sowie deren Mono- und Disalze auf anorganischer oder organischer Basis.

7. Verwendung nach einem der Ansprüche 1 bis 6, **dadurch gekennzeichnet, dass** das Derivat mit Thioetherfunktion, Sulfoxidfunktion oder Sulfonfunktion der Formel (I), (II) oder (III) unter Carboxymethylcystein und den Mono- und Disalzen dieser Verbindung auf anorganischer oder organischer Basis ausgewählt ist.

8. Verwendung mindestens eines Derivats mit Thioetherfunktion, Sulfoxidfunktion oder Sulfonfunktion der Formel (I), (II) oder (III) nach einem der Ansprüche 2 bis 7 zur topischen Anwendung als kosmetisches Mittel, das toxische Gase wie Ozon abfängt.

9. Verwendung eines Derivats mit Thioetherfunktion, Sulfoxidfunktion oder Sulfonfunktion der Formel (I), (II) oder (III) nach einem der Ansprüche 1 oder 3 bis 7 zur Herstellung einer Zusammensetzung für die topische Anwendung zum Schutz der Haut und der Keratinsubstanzen zur Vorbeugung, Abschwächung oder Unterdrückung der schädlichen Wirkungen von toxischen Gasen und organischen Verbrennungsrückständen.

10. Kosmetische Zusammensetzung nach einem der Ansprüche 2 bis 7, **dadurch gekennzeichnet, dass** das Derivat mit Thioetherfunktion, Sulfoxidfunktion oder Sulfonfunktion der Formel (I), (II) oder (III) in einer kosmetischen Zusammensetzung für die topische Anwendung zum Schutz der Haut und der Keratinfasern zur Vorbeugung, Abschwächung oder Unterdrückung der schädlichen Wirkungen von toxischen Gasen und organischen Verbrennungsrückständen verwendet wird.

11. Verwendung nach Anspruch 10, **dadurch gekennzeichnet, dass** die kosmetische Zusammensetzung 0,005 bis 10 % und vorzugsweise 0,1 bis 5 Gew.-% der Verbindung mit Thioetherfunktion, Sulfoxidfunktion oder Sulfonfunktion der Formel (I), (II) oder (III), bezogen auf das Gesamtgewicht der Zusammensetzung, enthält.

12. Verwendung nach Anspruch 10 oder 11, **dadurch gekennzeichnet, dass** die Zusammensetzung mindestens einen weiteren Anti-Pollution-Wirkstoff enthält.

13. Verwendung nach Anspruch 12, **dadurch gekennzeichnet, dass** der Anti-Pollution-Wirkstoff unter den Anthocyanen und/oder deren Derivaten, Ergothionin und/oder seinen Derivaten, Metallchelatbildnem, beispielsweise Derivaten von N,N'-Dibenzylethylendiamin-N,N'-diessigsäure, Antioxidantien, wie Ellagsäure, und zellulären Extrakten von Pflanzen aus der Familie der Pontederiaceae ausgewählt ist.

14. Verwendung nach einem der Ansprüche 10 bis 13, **dadurch gekennzeichnet, dass** die Zusammensetzung ferner ein kosmetisch akzeptables Medium enthält, das aus Wasser und/oder mindestens einem organischen Lösungsmittel besteht, das unter den hydrophilen organischen Lösungsmitteln, lipophilen organischen Lösungsmitteln, amphiphilen Lösungsmitteln oder deren Gemischen ausgewählt ist.

15. Verwendung nach Anspruch 14, **dadurch gekennzeichnet, dass** die organischen Lösungsmittel unter den mono- oder polyfunktionellen Alkoholen, gegebenenfalls ethoxylierten Polyethylenglykolen, Polypropylenglykolestern, Sorbit und seinen Derivaten, Dialkylisosorbiden, Glykolethern und Propylenglykolethern und Fettsäuren ausgewählt sind.

16. Verwendung nach Anspruch 14 oder 15, **dadurch gekennzeichnet, dass** das oder die organische(n) Lösungsmittel 5 bis 98 % des Gesamtgewichts der Zusammensetzung ausmachen.

17. Verwendung nach einem der Ansprüche 10 bis 16, **dadurch gekennzeichnet, dass** die Zusammensetzung ferner mindestens eine Fettphase enthält.

18. Verwendung nach Anspruch 17, **dadurch gekennzeichnet, dass** die Fettphase 0 bis 50 % des Gesamtgewichts der Zusammensetzung ausmacht.

19. Verwendung nach einem der Ansprüche 10 bis 18, **dadurch gekennzeichnet, dass** die Zusammensetzung ferner mindestens einen Zusatzstoff enthält, der unter den herkömmlichen wässrigen oder lipophilen Gelbildnern und/oder Verdickungsmitteln, hydrophilen oder lipophilen Wirkstoffen, Konservierungsmitteln, Antioxidantien, Parfums, Emulgatoren, Hydratisierungsmitteln, Pigmentierungsmitteln, Depigmentierungsmitteln, Keratolytika, Vitaminen, Emollientien, Maskierungsmitteln, grenzflächenaktiven Stoffen, Polymeren, Alkalisierungsmitteln oder Ansäuerungsmitteln, Füllstoffen, Radikalfängern für freie Radikale, Ceramiden, Sonnenschutzfiltern und insbesondere UV-Filtern, Insekten abwehrenden Wirkstoffen, schlanker machenden Wirkstoffen, Farbmitteln, Bakteriziden und Antischuppenmitteln ausgewählt ist.

20. Verwendung nach einem der Ansprüche 10 bis 19, **dadurch gekennzeichnet, dass** die Zusammensetzung als wässrige, wässrigalkoholische oder ölige Lösung, Öl-in-Wasser-Emulsion, Wasserin-Öl-Emulsion oder multiple Emulsion, wässriges oder öliges Gel, flüssiges, pastöses oder festes wasserfreies Produkt oder Dispersion eines Öls in einer wässrigen Phase mithilfe von Kügelchen vorliegt.

21. Verwendung nach einem der Ansprüche 10 bis 20, **dadurch gekennzeichnet, dass** die Zusammensetzung wie eine weiße oder farbige Creme, eine Pomade, eine Milch, eine Lotion, ein Serum, eine Paste, ein Schaum oder ein Feststoff aussieht.

22. Verwendung nach einem der Ansprüche 10 bis 21, **dadurch gekennzeichnet, dass** die Zusammensetzung einen pH-Wert von 3 bis 8 aufweist.

23. Kosmetisches Verfahren zum Schutz des Organismus gegen die schädlichen Wirkungen der Umweltverschmutzung, **dadurch gekennzeichnet, dass** es darin besteht, auf die Keratinsubstanz eine kosmetisch wirksame Menge mindestens eines Derivats mit Thioetherfunktion, Sulfoxidfunktion oder Sulfonfunktion der Formel (I), (II) oder (III) nach einem der Ansprüche 1 oder 3 bis 7 aufzutragen.

## Claims

1. Use for the preparation of a topical agent for protecting the skin and keratin materials so as to prevent, attenuate or eliminate the deleterious effects of toxic gases and organic compounds that are combustion residues, of at least one derivative containing a thioether, sulphoxide or sulphone function of formula (I), (II) or (III): 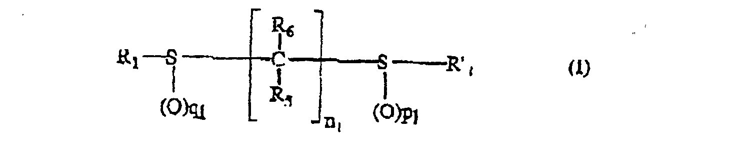 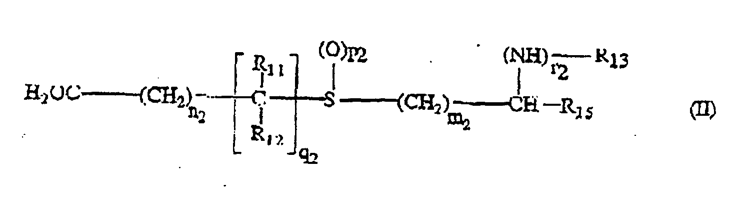 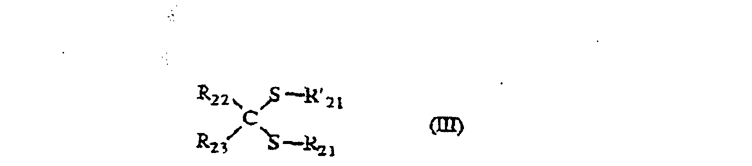 in which n₁ represents an integer ranging from 1 to 10, p₁ and q₁, which may be identical or different, represent 0, 1 or 2, n₂ represents an integer ranging from 0 to 5, p₂ represents 0, 1 or 2, q₂ represents 0, 1, 2 or 3, m₂ represents an integer ranging from 0 to 10, r₂ represents 0 or 1, q₂ and n₂ never both simultaneously being zero, R₁ and R'₁, which may be identical or different, represent a radical chosen from the groups:
a) 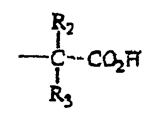
b) 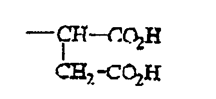
c) 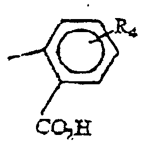
in which, R₂ and R₃, which may be identical or different, denote a hydrogen atom or a linear or branched C₁-C₈ alkyl or C₂-C₈ alkenyl group; R₄ denotes a hydrogen atom or a linear or branched C₁-C₈ alkyl group, a linear or branched C₂-C₈ alkenyl group or a linear or branched C₁-C₈ alkoxy group; R₅ and R₆ denote, independently of each other, a hydrogen atom, C₂-C₈ alkenyl, linear or branched C₁-C₈ alkoxy, phenyl, C₁-C₆ alkylcarboxylic or C₂-C₆ alkenylcarboxylic; R₁₁ and R₁₂ have the same meanings as R₅ and R₆; R₁₃ represents a linear or branched C₁-C₁₇ alkyl or C₂-C₁₇ alkenyl group or an acyl-CO-R₁₄ radical, with R₁₄ which denotes a linear or branched C₁-C₁₇ alkyl or C₂-C₁₇ alkenyl group; R₁₅ is chosen from a hydrogen atom, a linear or branched C₁-C₈ alkyl or C₂-C₈ alkenyl group, a C₁-C₅ alkylcarboxylic or C₂-C₅ alkenylcarboxylic group, or a -COOH function; R₂₁ and R'₂₁ denote groups of formulae: 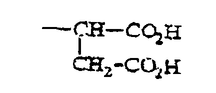 and
-CH₂-CO₂H
R₂₂ represents a group as follows:
i)
-CH₃
ii)
-CO₂H
iii) 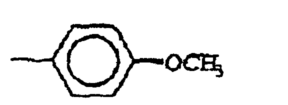
iv) 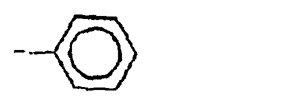
v) 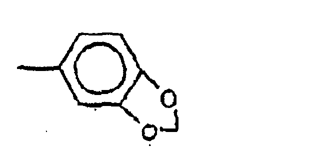
vi) 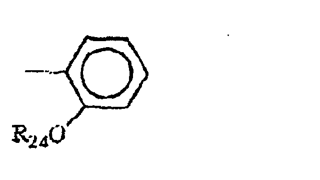
vii) 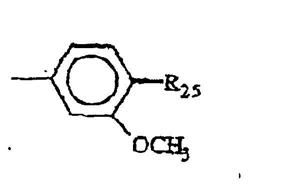
in which R₂₄ represents a group chosen from: -CH₂-COOH and -CO-CH₃ and R₂₅ represents a group chosen from: OH, OCH₃, OC(CH₃)₃, OCH₂COOH;
R₂₃ denotes a hydrogen atom, a linear or branched C₁-C₆ alkyl or C₂-C₆ alkenyl radical, or R₂₂ and R₂₃ together form an alkanediyl- (CH₂)m₃- group such that m₃ represents an integer ranging from 3 to 5, or R₂₂ and R₂₃ together form an aralkanediyl radical: 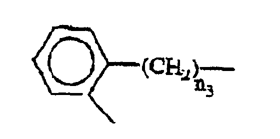 such that n₃ represents 3 or 4; or a salt thereof.

2. Cosmetic use of a derivative containing a thioether, sulphoxide or sulphone function of formula (I), (II) or (III) as defined in Claim 1, in topical application, as an agent for protecting the skin and keratin materials so as to prevent, attenuate or eliminate the deleterious effects of toxic gases and organic compounds that are combustion residues.

3. Use according to either of Claims 1 and 2, **characterized in that** the derivative containing a thioether function corresponds to formula (I) in which R₅ = R₆ = H, p₁ = q₁ and R₁ = R'₁.

4. Use according to either of Claims 1 and 2, **characterized in that** the derivative containing a sulphoxide function corresponds to formula (II) in which r₂ = 1, R₁₃ = H, R₁₅ = -COOH and m = 1 (cysteine derivatives) or m = 2 (homocysteine derivatives).

5. Use according to either of Claims 1 and 2, **characterized in that** the derivative containing a sulphone function corresponds to formula (III) in which R₂₂ is an aromatic radical, R₂₃ = H and R₂₁ = R'₂₁.

6. Use according to any one of Claims 1 to 5, **characterized in that** the derivative containing a thioether, sulphoxide or sulphone function is chosen from methylene-2,2'-dithiodibenzoic acid, ethylene-2,2'-dithiodibenzoic acid, ethylene-2,2'-disulphinyldibenzoic acid, 2,2'-(1,3-propanediyldithio)dibenzoic acid, 2,2'-(1,4-butanediyldithio)dibenzoic acid, 2,2'-(1,5-pentanediyldithio)dibenzoic acid, 2,2'-(1,6-hexanediyldithio)dibenzoic acid, 2,2'-(1,6-hexanediyldisulphinyl)dibenzoic acid, α,α'-(1,6-hexanediyldithio)dibenzoic acid, 2,2'-(1,8-octanediyldithio)dibenzoic acid, 2,2'-(1,10-decanediyldithio)dibenzoic acid, α,α'-(1,10-decanediyldithio)dibenzoic acid, methylene-α,α'-dithiodiacetic acid, ethylene-α,α'-dithiodiacetic acid, ethylene-α,α'-disulphinyldiacetic acid, ethylene-α,α'-disulphonyldiacetic acid, α,α'-(1,6-hexanediyldithio)-diacetic acid, α,α'-(1,6-hexanediyldisulphinyl)diacetic acid, α,α'-(1,6-hexanediyldisulphonyl)diacetic acid, α,α'-(1,10-decanediyldithio)diacetic acid, α,α'-(1,10-decanediyldisulphinyl)diacetic acid, α,α'-(1,10-decanediyldisulphonyl)diacetic acid, S-(carboxymethyl)-cysteine, S-(carboxyethyl)cysteine, S-(4-carboxybutyl)-cysteine, S-(carboxymethyl)sulphinyl cysteine, S-(carboxymethyl)sulphonyl cysteine, S-(1-carboxy-1-ethyl)cysteine, S-(2-carboxy-2-propyl)cysteine, α,α'-(p-methoxybenzylidenedithio)disuccinic acid, α,α'-(benzylidenedithio)disuccinic acid, α,α'-(piperonylidenedithio)disuccinic acid, α,α'-(vanillylidenedithio)disuccinic acid, α,α'-(veratrylidenedithio)-disuccinic acid, α,α'-(4-butoxy-3-methoxybenzylidenedithio)disuccinic acid, α,α'-(4-carboxymethyloxy-3-methoxybenzylidenedithio)disuccinic acid, α,α'-(p-methoxybenzylidenedithio)diacetic acid, α,α'-(benzylidenedithio)diacetic acid, α,α'-(piperonylidenedithio)diacetic acid, α,α'-(vanillylidenedithio)-diacetic acid, α,α'-(veratrylidenedithio)diacetic acid, and also the mono-salts and di-salts thereof with a mineral or organic base.

7. Use according to any one of Claims 1 to 6, **characterized in that** the derivative containing a thioether, sulphoxide or sulphone function of formula (I), (II) or (III) is chosen from carboxymethylcysteine and also the mono-salts and di-salts thereof with a mineral or organic base.

8. Use in topical application of at least one derivative containing a thioether, sulphoxide or sulphone function of formula (I), (II) or (III) as defined in one of Claims 2 to 7, as a cosmetic agent for trapping toxic gases such as ozone.

9. Use of a derivative containing a thioether, sulphoxide or sulphone function of formula (I), (II) or (III) as defined in any one of Claims 1 or 3 to 7, in the preparation of a topical composition for protecting the skin and keratin materials so as to prevent, attenuate or eliminate the deleterious effects of toxic gases and organic compounds that are combustion residues.

10. Cosmetic use according to one of Claims 2 to 7, **characterized in that** the derivative containing a thioether, sulphoxide or sulphone function of formula (I), (II) or (III) is used in a cosmetic composition for topical application for protecting the skin and keratin materials so as to prevent, attenuate or eliminate the deleterious effects of toxic gases and organic compounds that are combustion residues.

11. Use according to Claim 10, **characterized in that** said cosmetic composition contains from 0.005% to 10% and preferably from 0.1% to 5% by weight of compounds containing a thioether, sulphoxide or sulphone function of formula (I), (II) or (III) relative to the total weight of the composition.

12. Use according to Claim 10 or 11, **characterized in that** said composition also contains at least one other antipollution compound.

13. Use according to Claim 12, **characterized in that** said antipollution compound is chosen from anthocyans and/or derivatives thereof, ergothioneine and/or its derivatives, metal-chelating agents, for instance N,N'-dibenzylethylenediamine-N,N'-diacetic acid derivatives, antioxidants, for instance ellagic acid, and cell extracts of plants from the Pontederiacea family.

14. Use according to any one of Claims 10 to 13, **characterized in that** the composition also contains a cosmetically acceptable medium consisting of water and/or of at least one organic solvent chosen from the group consisting of hydrophilic organic solvents, lipophilic organic solvents and amphiphilic solvents, or mixtures thereof.

15. Use according to Claim 14, **characterized in that** the organic solvents are chosen from the group consisting of monofunctional or polyfunctional alcohols, optionally oxyethylenated polyethylene glycols, polypropylene glycol esters, sorbitol and its derivatives, dialkyl isosorbides, glycol ethers and polypropylene glycol ethers, and fatty esters.

16. Use according to Claim 14 or 15, **characterized in that** the organic solvent(s) represent(s) from 5% to 98% relative to the total weight of the composition.

17. Use according to any one of Claims 10 to 16, **characterized in that** the composition also comprises at least one fatty phase.

18. Use according to Claim 17, **characterized in that** the fatty phase represents from 0 to 50% relative to the total weight of the composition.

19. Use according to any one of Claims 10 to 18, **characterized in that** the composition also contains at least one additive chosen from the group consisting of standard aqueous or lipophilic gelling agents and/or thickeners, hydrophilic or lipophilic active agents, preserving agents, antioxidants, fragrances, emulsifiers, moisturizers, pigmenting agents, depigmenting agents, keratolytic agents, vitamins, emollients, sequestering agents, surfactants, polymers, acidifying or basifying agents, fillers, free-radical scavengers, ceramides, sunscreens, especially ultraviolet screening agents, insect repellents, slimming agents, colorants, bactericides and antidandruff agents.

20. Use according to any one of Claims 10 to 19, **characterized in that** the composition is in the form of an aqueous, aqueous-alcoholic or oily solution, an oil-in-water or water-in-oil or multiple emulsion, an aqueous or oily gel, a liquid, pasty or solid anhydrous product or a dispersion of oil in an aqueous phase with the aid of spherules.

21. Use according to any one of Claims 10 to 20, **characterized in that** the composition has the appearance of a white or coloured cream, an ointment, a milk, a lotion, a serum, a paste, a mousse or a solid.

22. Use according to any one of Claims 10 to 21, **characterized in that** the composition has a pH of between 3 and 8.

23. Cosmetic process for protecting the body against the effects of pollution, **characterized in that** it consists in applying to keratin material a cosmetically effective amount of at least one derivative containing a thioether, sulphoxide or sulphone function of formula (I), (II) or (III) as defined in one of Claims 1 or 3 to 7.
